(19)

Europäisches
Patentamt
European
Patent Office
Office européen
des brevets

(11) **EP 1 949 857 B1**

(12) **EUROPEAN PATENT SPECIFICATION**

(45) Date of publication and mention
of the grant of the patent:
**13.06.2012 Bulletin 2012/24**

(21) Application number: **06832658.6**

(22) Date of filing: **15.11.2006**

(51) Int Cl.:
*A61B 8/08* (2006.01)          *A61B 8/14* (2006.01)
*G06T 7/00* (2006.01)

(86) International application number:
**PCT/JP2006/322727**

(87) International publication number:
**WO 2007/058195 (24.05.2007 Gazette 2007/21)**

(54) **ULTRASONOGRAPHIC DEVICE**

ULTRASCHALLGERÄT

DISPOSITIF ULTRASONOGRAPHIQUE

(84) Designated Contracting States:
**DE FR GB IT NL**

(30) Priority: **15.11.2005  JP 2005330523**

(43) Date of publication of application:
**30.07.2008  Bulletin 2008/31**

(73) Proprietor: **Hitachi Medical Corporation
Tokyo 101-0021 (JP)**

(72) Inventor: **CHONO, Tomoaki
Tokyo 101-0021 (JP)**

(74) Representative: **Buchetmann, Dominik et al
Strehl Schübel-Hopf & Partner
Maximilianstrasse 54
80538 München (DE)**

(56) References cited:
WO-A1-03/075770          JP-A- 08 308 834
JP-A- 2002 140 689       JP-A- 2002 253 539
JP-A- 2003 275 204       JP-A- 2005 049 968
JP-A- 2005 270 421       US-A1- 2003 045 796
US-A1- 2005 033 123      US-A1- 2005 238 216

• TAKAGI M. ET AL.: 'Shinpen Gazo Kaiseki
Handbook', 10 September 2004 pages 1844 -
1846, XP003009060

**Description**

Technical Field

The present invention relates to an ultrasonographic device, and particularly to an improvement of an ultrasonographic device having a function of matching tomogram information of an examinee obtained through an ultrasonic probe with diagnosis image information taken from a database and displaying a matching result on a display device to support and assist subsequent measurement, diagnosis of the examinee.

Background Art

It is very useful for diagnosis of pathologic lesion of heart to quantitatively estimate, for example, a blood flow, the volume of a heart chamber and strain of heart muscle in diagnosis of cardiac functions by using an ultrasonographic device.

Therefore, when the diagnosis of the cardiac functions is performed by using an ultrasonographic device, tomograms of a heart are obtained from various directions through an ultrasonic probe and these tomograms are observed and measured. For example, a valve, the wall thickness of heart muscle, and a wall motion can be observed on a short-axis view, and a valve, the volume of a heart chamber, the blood flow, the wall thickness of heart muscle, and the wall motion can be obtained on a long-axis view.

In a case where the diagnosis of the cardiac functions is performed while a tomogram as described above is observed, if the type of such a view displayed on a display device can be easily identified, for example, if it can be identified whether the view is a short-axis view or a long-axis view or the like, the subsequent measurement and diagnosis are extremely efficient.

Therefore, for example, JP-A-2002-140689 discloses, for example, a medical image processing device used in combination with the ultrasonographic device in which cross-sectional image analyzing means for storing image patterns of respective cross-sectional images of a living organ, particularly a heart as dictionary images compares a two-dimensional cross-sectional image obtained by obtaining means with the stored dictionary images, cross-sectional information comprising the three-dimensional cross-sectional position and cross-sectional direction of the cross-sectional image concerned is analyzed on the basis of a dictionary image having a similar pattern, an analysis result is output to output means, and the cross-sectional position and direction are automatically recognized from the obtained cross-sectional image data of the living body, so that structural information of a target object which will be an aid for diagnosis of a doctor can be extracted.

However, the analysis of the cross-sectional information in a medical image processing device used for the ultrasonographic device disclosed in JP-A-2002-140689 is based on the assumption that an examiner can first obtain a tomogram proper to measurement, and it deals with the analysis of the cross-sectional information after the proper tomogram is obtained.

Therefore, in the cardiac function measurement, the examiner is required to select a measurement item from a large number of measurement menus in accordance with a measurement purpose before a measurement is carried out, and also required to carry out an examination while checking it on the basis of examiner' s experience whether a proper view matched with the measurement purpose can be obtained.

An object of the present invention is to provide an ultrasonographic device that can automatically identify the type of a view obtained by an examiner on a real-time basis.

Disclosure of the Invention

The above object is net by the ultrasonographic device defined in claim 1. The dependent claims relate to preferred embodiments.

Illustrative examples and embodiments of the invention will be briefly described hereunder.

(1) An ultrasonographic device may comprise, for example: a template conversion unit for converting tomogram information of an examinee to tomogram information which is formed into a template; a database for storing diagnosis image information formed into a template in advance; a matching unit for matching the templated tomogram information of the examinee with the templated diagnosis image information; and a display device for displaying a matching result of the matching unit.

(2) An ultrasonographic device is, for example, based on the construction of (1), and characterized in that when the matching between the templated tomogram image and the templated diagnosis image information is satisfied by the matching unit, a symbol mark such as a body mark or a character which represents the type of the tomogram information is displayed on the display device together with a tomogram image thereof.

(3) An ultrasonographic device is, for example, based on the construction of (1), and characterized in that templated diagnosis image information taken from the database contains not only standard diagnosis image information of an able-bodied person, but also diagnosis image information containing a lesioned part, and when the matching between the templated tomogram information and the diagnosis image information containing the lesioned part is satisfied by the matching unit, a disease name is displayed on the display device together with the tomogram image thereof.

(4) An ultrasonographic device is, for example, based on the construction of (1), and characterized in that tomogram information of an examinee obtained through an ultrasonic probe is tomogram information of a heart which is obtained in synchronization with some time phase of an electrocardiogram of the examinee.

According to the present invention, it can be checked on a real-time basis that a proper view matched with a measurement purpose can be obtained, so that the precision of the measurement based on the check concerned is enhanced, and the efficiency of a subsequent examining diagnosis is drastically enhanced.

Brief Description of Drawings

Fig. 1 is a block diagram showing an embodiment of an ultrasonographic device according to the present invention.
Fig. 2 is a flowchart showing the procedure of obtaining a tomogram of the heart of an examinee according to an embodiment of the present invention as shown in Fig. 1, identifying the tomogram and measuring the identified tomogram.
Fig. 3 is a diagram showing signal obtaining of electrocardiogram (ECG) R-wave synchronization as shown in Fig. 2 and subsequent identification processing.
Fig. 4 is a diagram showing an example of a signal which is extracted in a signal value extracting unit 3 of Fig. 1 and input to a template conversion unit 4.
Fig. 5 is a diagram showing another example of the signal which is likewise extracted in the signal value extracting unit 3 of Fig. 1 and input to the template conversion unit 4.
Fig. 6 is a diagram showing another example of the signal which is likewise extracted in the signal value extracting unit 3 of Fig. 1 and input to the template conversion unit 4.
Fig. 7 is a diagram showing a display example in which a tomogram image displayed on the display device 9 of Fig. 1 and the type thereof are represented by a body mark and a character.
Fig. 8 is a diagram showing a method of calculating the speed of a heart motion when the speed of the heart motion is utilized as a time phase in place of the signal obtaining of the R-wave synchronization described with reference to Fig. 3.

Best Modes for carrying out the Invention

An embodiment of an ultrasonographic device according to the present invention will be described hereunder with reference to the drawing.

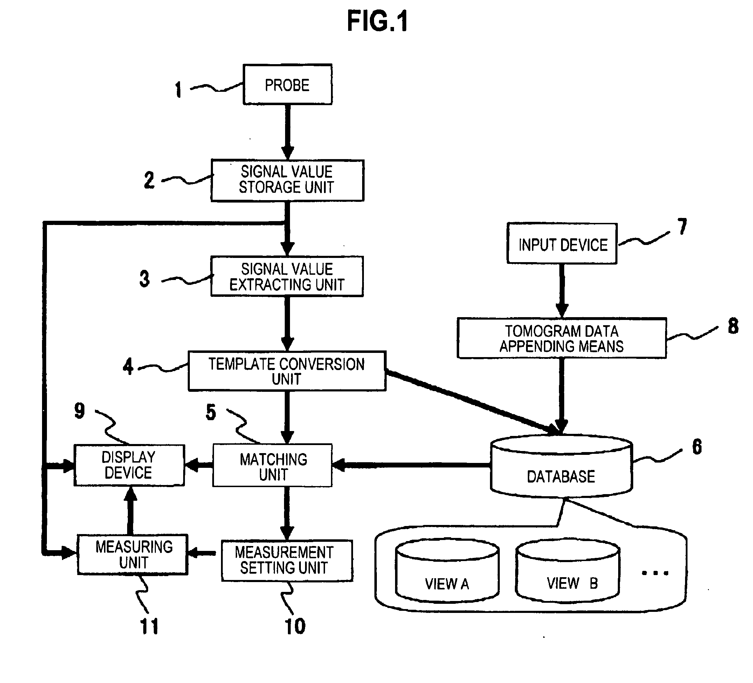

Fig. 1 is a block diagram showing an embodiment of the ultrasonographic device according to the present invention. The ultrasonographic device according to this embodiment is constructed so that it can take in diagnosis image information from a database 6. The database 6 may be constructed in a storage device equipped to the ultrasonographic device itself, or it may be disposed so as to be spaced from the ultrasonographic device as a separate body from the ultrasonographic device.

Diagnosis image information is stored into the database 6 with being -successively passed through an input device 7 containing a mouse, a keyboard, a track ball and a template data reading unit from a disk or the like, and tomogram data appending means 8 for adding relevant information. Here, the diagnosis image information stored in the database 6 is constructed as so-called templated diagnosis image information formed. As descried later, the diagnosis image information is correlated with tomogram information of an examinee obtained through an ultrasonic probe 1 on a real-time basis. The templated diagnosis information is input not only from the input device 7, but also from a template converting unit 4 described later which is equipped in the ultrasonographic device as occasion demands. This is because the latest templated information which is as much as possible is stored in the database 6. As a method of creating a template is used as an eigen space method, a subspace method, a mutual subspace method or the like.

Furthermore, with respect to each diagnosis image information, the tomogram data appending unit 8 appends other data such as the type of the diagnosis image information, etc. in association with the diagnosis image information. Accordingly, when one of the diagnosis image information is selected, various kinds of data associated with the diagnosis image information, for example, the type name of the image, the age, sex and disease name of an examinee, a standard measurement initial setting value of the image and measurement set values which were past set by the user can be obtained.

A set which is matched with the measurement and the diagnosis purpose is prepared as an electronic file for the database 6. The content of the set matched with the purpose is an assembly containing the parasternal view (left ventricular long-axis view, left ventricular short-axis view, right ventricular inflow path long-axis cross-section) or the cross-sections of an apical site approach image (left ventricular long-axis cross-section, four-chamber cross-section, five-chamber cross-section, two-chamber cross-section) which are obtained by a normal examination for the purpose of identifying the type of the cross-section, an assembly containing tomograms of hypertrophic cardiomyopathy (containing various hypertropic styles), dilated cardiomyopathy, etc. which are obtained for the purpose of identifying the type of cardiomyopathy, an assembly containing tomograms of mitral valve, aortic valve disease, tricuspid valve disease, etc. which are obtained for the purpose of identifying the type of valve disease, and an assembly containing tomograms of the interatrial septum defect, the interventricular septum defect, etc. which are obtained for the purpose of identifying the type of congenital cardiac disease, for example.

These sets may be used independently of one another, or these sets may be integrated into one set so that identification of the view and identification of the type are performed at the same time. Furthermore, various kinds of templated diagnosis image information which correspond to the sex and age of a patient can be prepared every display depth or view, whereby a database to identify an input tomogram of a broad range can be prepared. If the set of the database is set so as to contain some specific disease data, it can be used to identify a disease. For example, if the database of dilated cardiomyopathy and hypertrophic cardiomyopathy is prepared, it is effective to identify a lesion or a disease from tomograms obtained when the shape of a heart is different from a normal one. Furthermore, the user can create a new database set based on the viewpoint at the user side, and add/delete data to/from the database set, thereby constructing a database corresponding to the use situation of the user.

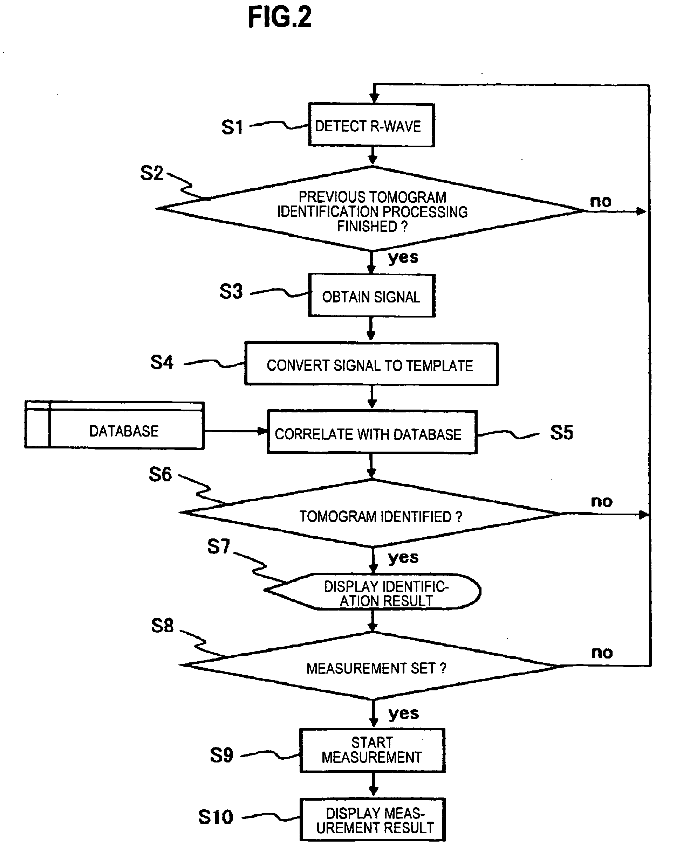

. The ultrasonographic device is equipped with an ultrasonic probe 1 used in contact with the body surface of the examinee or the like, and a signal value storage unit 2 for storing tomogram information (signal) of the examinee which is created by a reflection echo signal in the examinee obtained through the ultrasonic probe 1. With respect to the tomogram information, as is apparent from the description made later with reference to Fig. 2, tomograms of a living organ of an examinee, for example, the tomograms of a moving heart are targeted, and (for example) the tomograms are obtained every detection of R-wave.

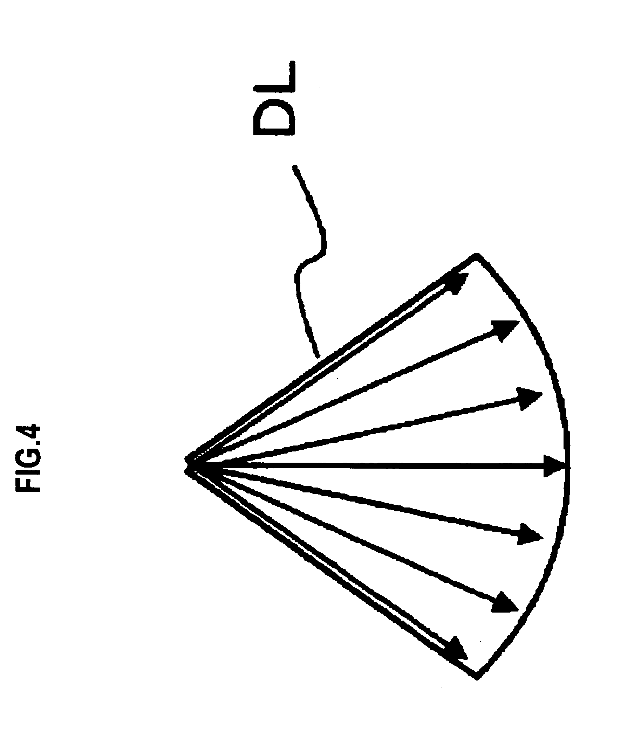

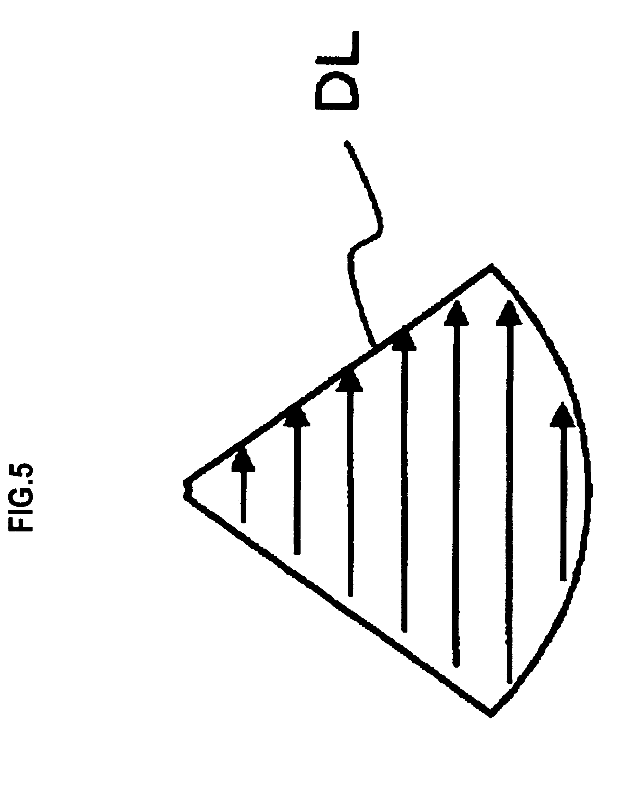

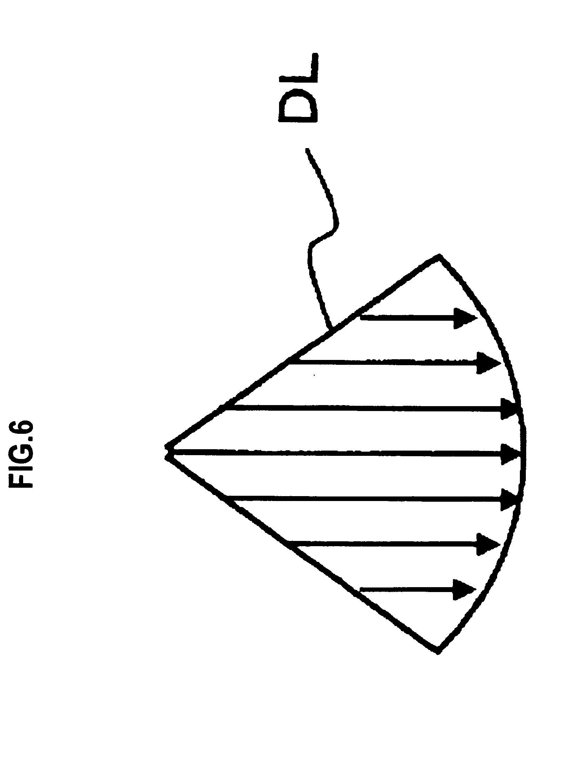

The ultrasonographic device is equipped with a signal value extracting unit 3 for extracting prescribed tomogram information from the tomogram information (signal) stored in the signal value storage unit 2. The tomogram information to be extracted may be the directly extracted amplitude value of the information, or extracted by calculating a static amount in any area. For example, when a sector type ultrasonic probe is used as the ultrasonic probe 1, all echo signals of a tomogram plane DL constituting a fan, or the brightness value of an image may be extracted. Furthermore, signals at respective positions which are scanned in fan-like fashion on the tomogram plane DL may be extracted as shown in Fig. 4, signals at respective positions which are scanned in the lateral direction on the tomogram plane DL may be extracted as shown in Fig. 5, or signals at respective positions which are scanned in the vertical direction on the tomogram plane DL may be extracted as shown in Fig. 6. Furthermore, when the resolution of an image is high and thus the data amount is excessively large, the resolution may be lowered or pixels may be thinned out to extract information.

When the signals at the respective positions scanned in a fan-like fashion on the tomogram plane DL as shown in Fig. 4 are extracted, signal values are taken out in the same direction as ultrasonic beams, and thus there is an effect that the echo signal values can be taken out at high speed. Conversely, the density is high at a shallow portion (near to the probe), and the density is low at a deep portion (the line density is higher at an upper portion and the line density is lower at a lower portion as shown in Fig. 4), and the amount of information is unbalanced in accordance with the depth when the signal values of the overall heart are extracted. However, when the signals are extracted by lateral or vertical scanning as shown in Figs. 5 and 6, there is an effect that the density of information is equal between the shallow portion and the deep portion because the signal values are obtained at the lattice coordinates, so that the information of the overall heart can be extracted with excellent balance.

The tomogram information extracted by this signal value extracting unit 3 is converted to templated tomogram information by the template conversion unit 4. By creating the templated tomogram information as described above, the matching with the corresponding templated diagnosis image information from the database can be easily performed in an extremely short time.

As a templating method, when the amount of data is reduced or a feature portion is extracted, the eigen space method as described below may be used, and when the signal values of plural time phases are obtained, the subspace method may be used. The tomogram information which is templated according to the predetermined method as described above is templated in a predetermined registerable form, and stored in the database 6 as occasion demands.

Here, the method of obtaining tomogram information templated in a registerable form in the database 6 by using the eigen space method will be described.

First, a set of signal value vectors of tomogram information obtained from the signal value extracting unit 3 is represented by A. The number of tomograms used for templating is represented by N, and the average value ($\overline{A}$) and covariance matrix S thereof are calculated according to the following equation to create the eigen space. T represents transposition.

$$\overline{A} = \frac{1}{N}\sum_{n=1}^{N} A_n \qquad \ldots (1)$$

$$S = \frac{1}{N}\sum_{n=1}^{N}(A_n - \overline{A})(A_n - \overline{A})^T \qquad \ldots (2)$$

S is subjected to eigen value decomposition to calculate eigen values $\lambda$ and eigenvectors u. The eigen values $\lambda$ are arranged in descending order, and an eigenvector $\Phi = \{u_1, u_2, ..., u_k\}$ corresponding to any k is determined from the largest eigen value, and a database is complied by setting as a template a vector A* obtained by projecting A in the following equation (3). Accordingly, the capacity of the database is reduced, and also a database of only the feature serving as a main part of the signal can be efficiently made.

$$A^* = \Phi^T(A - \overline{A}) \qquad \ldots (3)$$

Furthermore, a method using the subspace method is known as another method of templating so as to be registerable in the database 6. By using signal values of L frames (for example, selected from P, Q, R,S, T, U waves on ECG or another frame) with respect to a signal value vector Ap of some cross-section p, the average value (Ap) and the covariance matrix Sp are calculated from the following equations to create the subspace.

$$\overline{A}_p = \frac{1}{L}\sum_{n=1}^{L} A_{pn} \qquad \ldots (4)$$

$$S_p = \frac{1}{L}\sum_{n=1}^{L}(A_{pn} - \overline{A}_p)(A_{pn} - \overline{A}_p)^T \qquad \ldots (5)$$

Sp is subjected to eigen value decomposition to calculate eigen values $\lambda_P$ and eigenvectors up. The eigen values are arranged in descending order, and an eigenvector $\Phi_p = \{u_{p1}, u_{p2}, ..., U_{pk}\}$ corresponding to any k is determined from the largest eigen value, and a database is complied by setting as a template a vector $A^*_p$ obtained by projecting A in the following equation (6). Accordingly, the capacity of the database is reduced, and also a database of only the feature serving as a main part of the image can be efficiently made. Furthermore, the signal varies at all times, and thus a feature which cannot be obtained by only a single signal can be stably extracted by using plural signals.

$$A^*p = \Phi p^T(A - \overline{A}p) \qquad \ldots (6)$$

A signal value vector $A_{new}$ in which the type of the cross-section input from the template conversion unit 4 for new matching is unknown, and the database in which cross-section types are known are subjected to the matching processing in a matching unit 5. In this processing, the cross-section having the nearest feature to the feature of the signal value vector $A_{new}$ of the input cross-section is found out from the diagnosis image information of the database 6.

When the database 6 templated by using the eigen space method, $A_{new}$ is projected to the eigen space as indicated in the following equation (7).

$$A *_{new} = \Phi^{T}(A_{new} - \overline{A}) \qquad \dots \quad (7)$$

The difference between the projected vector A*$_{new}$ and A* of the equation (3) is quantified, and the view having the smallest difference is found out. The type of the view having the smallest difference is set as the identified view type. A scale such as Euclidean distance, Mahalanobis distance, Manhattan distance, degree of similarity or the like may be used for the calculation of this difference. The above scale is used when the difference is quantified.

When the database 6 templated by using the subspace method is used, A$_{new}$ is projected to the subspace of each view p as indicated by the equation (8).

$$A *_{pnew} = \Phi^{T}_{p}(A_{new} - \overline{A}_{p}) \qquad \dots \quad (8)$$

The difference between the projected vector A*$_{pnew}$ and A*$_{p}$ of the equation (6) is quantified to identify the view.

In both the methods, a threshold value is provided with respect to the difference between the templated input and the template in the database, and if the difference is larger than the threshold value, the matching is set to be impossible.

Furthermore, the mutual subspace method of inputting plural image signals, creating the templated input subspace and matching the input subspace and the subspace of the database may be used.

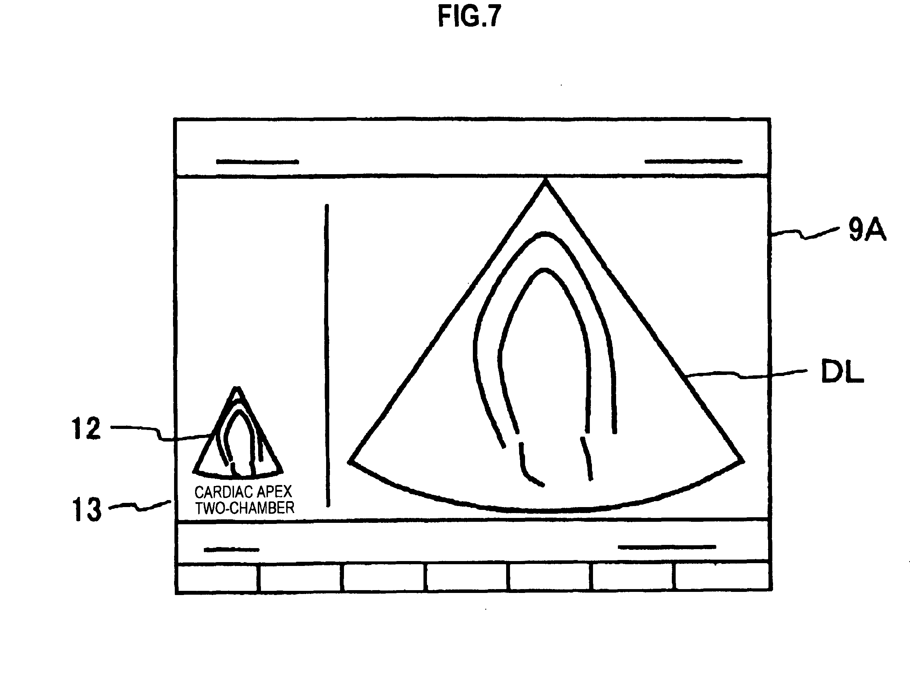

As described above, the templated tomogram of the examinee obtained through the ultrasonic probe 1 is correlated to the templated diagnosis image information taken from the database 6 in the matching unit 5, and when the difference therebetween is not more than a predetermined threshold value, a body mark 12 as a symbol mark representing the type of the tomogram information and a character 13 are displayed on the display plane 9A of the display device 9 as shown in Fig. 7. The type of the tomogram image information contains "cardiac apex two-chamber", "parasternal short-axis", "parasternal long-axis" "the interatrial septum defect" or the like, for example. In this case, one of the body mark 12 and the character 13 may be displayed. In a most area of the display plane 9A of the display device 9, the tomogram plane DL which is the non-templated tomogram information of the examinee obtained through the ultrasonic probe 1 is displayed, and the type of this tomogram is set to be immediately identified by the body mark 12 or the character 13.

In this case, when diagnosis image information containing disease data is prepared as diagnosis image information stored in the database 6, a disease may be displayed on the display plane 9A. Furthermore, when it is impossible to correlate the tomogram image obtained through the ultrasonic probe 1 with the diagnosis image information taken from the database 6, a mark indicating "matching-impossible" may be displayed or non-display may be made. As the display of the body mark 12 and the character 13, the matching result may be renewed every R-wave is detected, or may be renewed every time the matching processing is completed. Accordingly, the examiner can check whether the tomogram matched with the examination purpose can be correctly obtained.

A measurement setting unit 10 renews the measurement menu and the initial set value on the basis of the identified view type. For example, when a short-axis view is identified, measurement items used for only the long-axis view are deleted, and a menu is constructed by only measurement items used for the short-axis view. When the examiner enters the measurement mode, only the short-axis menu is displayed. Accordingly, the load imposed on the cumbersome measurement item selection from all the measurement items like the prior art can be reduced. The same operation is also carried out when another view is identified. Furthermore, measurement set values which are made in the form of a database as a set with the identified views may be called and automatically input as the initial set values.

When the set value of the measurement item is set in the measurement set unit 10, a measurement is executed on the tomogram displayed on the basis of the set value in the measuring unit 11, and the result thereof is displayed on the display device 9.

Fig. 2 is a flowchart showing the operation procedure of obtaining tomograms of the heart of an examinee and measuring the identified tomograms by the ultrasonographic device according to the embodiment of the present invention shown in Fig. 1.

In this embodiment, the templated diagnosis image information stored in the database 6 contains information created at some time phase of an electrocardiogram (ECG), for example, in synchronization with R-wave, and in the matching described later, information created in synchronization with the corresponding time phase is read out. The diagnosis image information stored in the database 6 may be only information which is created at some time-phase, for example, in synchronization with the R-wave. When only templates at some predetermined time phase are stored in the database 6, the storage capacity of the database 6 can be reduced as compared with a style in which moving pictures at all time phases are stored, and the reading time is shorter, so that the matching time can be also shortened.

First, R-wave is detected from the examinee in step S1. A reflection echo signal for creating a tomogram is taken in synchronization with the R-wave. In step S2, it is judged whether the identification processing of a previous tomogram is finished or not. The identification processing is required to be finished during one beat of the heart. However, the length of one beat is different among individuals, and if the identification processing is not finished during one beat, the identification processing may be extended until next R-wave is detected. This is because the tomogram is necessarily created in synchronization with the R-wave. If the previous identification processing is finished in step S2, the reflection echo signal is obtained in step S3. In step S4, tomogram information which is templated in such a form that it can be correlated with templates in the database 6 is obtained from the obtained signal.

Then, in step S5, the templated diagnosis image information is taken from the database 6, and correlated with the templated tomogram information. In step S6, it is judged whether the tomogram information is coincident with the diagnosis image information, that is, it is judged whether the tomogram information is identified or not. If it is not identified, the processing waits until next R-wave detection, and the above operation is repeated. If the tomogram image information is identified in step S6, the identification result is displayed on the display device 9 in step S7.

Subsequently, it is judged whether the measurement setting is carried out through the measurement setting unit 10 in accordance with the type of the identified tomogram by the examiner in step S8. If it has not yet been identified, the above operation is repeated. If the measurement setting is carried out, the measurement of the tomogram displayed on the display device is executed according to the setting in step S9, and the measurement result is displayed on the display device 9 in step S10.

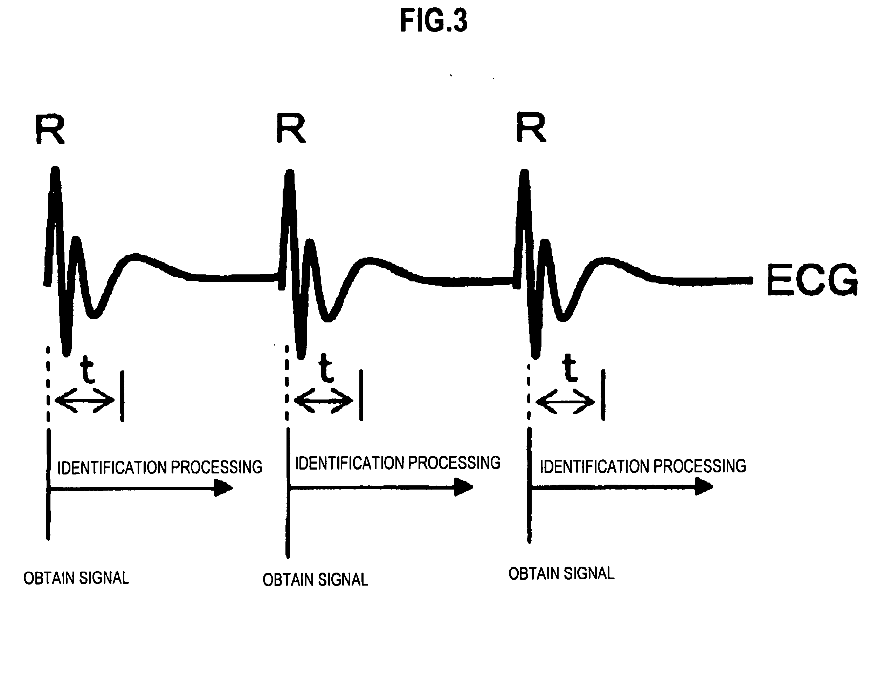

Fig. 3 is a diagram showing the identification processing and the signal obtaining timing when the identification processing (the processing from step S3 to step S6 of Fig. 2) is executed during the time period from the detection of R-wave till the detection of the next R-wave, in the operation shown in Fig. 2. In this case, the signal is obtained on the basis of the detection of the R-wave. However, for example when the image information templating operation based on the subspace method requiring to obtain signals at plural time phases is carried out, in addition to the signal acquisition in synchronization with the R-wave, signal acquisition may be carried out after a fixed time t elapses from the R-wave, for example.

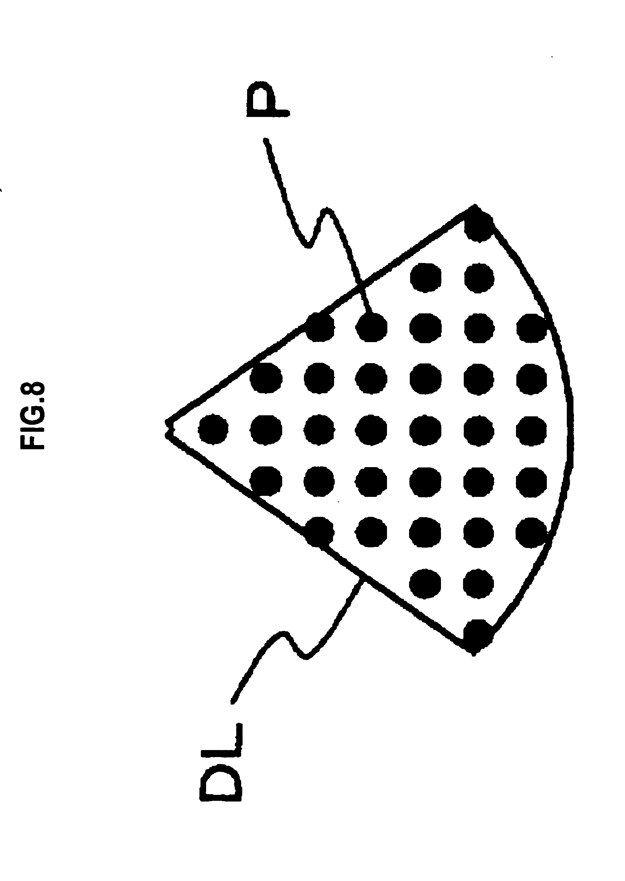

In the above embodiment, the electrocardiogram (ECG) based on the electrocardiograph can be prepared. However, for example, when the electrocardiogram (ECG) cannot be immediately prepared like an emergency case or the like, the time phase on ECG is estimated from the motion of the heart. The moving speed of the heart is not fixed during one beat. Accordingly, it is possible that the moving speed is measured, the time phase at a specific speed, for example, at the highest speed or the lowest speed is detected and the signal is taken in synchronization with the time phase. In this case, in order to measure the moving speed of the heart, a method called as optical flow may be used, for example. That is, as shown in Fig. 8, many points (measurement points) P for measuring the speed are set on the overall tomogram plane DL on which the heart is displayed, the speeds of the measurement points P are calculated by the optical flow method, and the moving speed of the overall heart is calculated from the statistic amount of these speeds.

**Claims**

1. An ultrasonographic device comprising:

   a database (6) storing templated diagnosis image information based on various viewpoints of a living organ of an examinee, said diagnosis image information having been templated by a predetermined method;
   a template conversion unit (4) for templating, by said predetermined method, tomogram image information of the living organ constructed by echo signals collected through an ultrasonic probe (1) brought into contact with the examinee so that the templated tomogram image information can be correlated with the templated diagnosis image information stored in the database (6);
   a matching unit (5) for matching the tomogram image information templated by the template conversion unit (4) with the templated diagnosis image information stored in the database (6); and
   a display device (9) for displaying a matching result of the matching unit (5) in real-time together with tomograms of the living organ constructed by the echo signals collected through the ultrasonic probe (1) brought into contact with the examinee,
   **characterised in that**
   said predetermined method includes projecting a signal value vector ($A$; $A_p$) of the diagnosis or tomogram image information to an eigen space or a subspace to obtain a template vector ($A^*$, $A^*_{new}$; $A^*_p$, $A^*p_{new}$), and
   said matching unit (5) is adapted to determine as the matching result the viewpoint with the template vector ($A^*$; $A^*_p$) in the stored templated diagnosis image information which has the smallest difference to the template vector ($A^*_{new}$; $A^*_{pnew}$) obtained from the tomogram image information.

2. The device of claim 1, wherein the living organ of the examinee is a heart, and the tomogram image information thereof is obtained in synchronization with a predetermined time phase on an electrocardiographic complex of the examinee.

3. The device of claim 2, wherein the matching unit (5) is adapted to read out the templated diagnosis image information corresponding to a predetermined time phase from the database (6).

4. The device of claim 3, wherein the templated diagnosis image information stored in the database (6) is only the templated diagnosis image information corresponding to the predetermined time phase.

5. The device of claim 2, further comprising an identification processing judging unit for judging whether an identification processing of a previous tomogram is finished or not.

6. The device of claim 1, wherein a part of the tomogram image information templated in the template conversion unit (4) is made in such a form that it can be registered in the database (6).

7. The device of claim 6, wherein a signal input to the template conversion unit (4) is a signal value from each point which is scanned along a direction of an ultrasonic beam from the ultrasonic probe (1) on a tomogram.

8. The device of claim 6, wherein a signal input to the template conversion unit (4) is a signal value from each point which is scanned in a vertical or lateral direction on a tomogram plane (DL).

9. The device of claim 6, wherein the tomogram image of the heart is obtained in synchronization with the time phase of the moving speed of the heart which is associated with a predetermined time phase on an electrocardiographic complex of the examinee.

10. The device of claim 1, wherein when the matching between the tomogram image information templated in the matching unit (5) and the templated diagnosis image information stored in the database (6) is satisfied in the matching unit (5), a symbol mark (12) or a character (13) representing the type of the tomogram image information is displayed as a matching result on the display device (9).

11. The device of claim 1, wherein
templated diagnosis image information containing a lesion site is stored in the database (6) in addition to standard templated diagnosis image information of an healthy person, and
when the matching between templated tomogram information and the templated diagnosis image information containing the lesion site stored in the database (6) is satisfied in the matching unit (5), a disease name of the tomogram information is displayed as a matching result on the display device (9).

12. The device of claim 1, wherein templated diagnosis image information stored in the database (6) is appended with appendant information containing the age, sex and disease name of the examinee, a measurement initial set value and past measurement set value which are associated with the templated diagnosis image information.

**Patentansprüche**

1. Ultraschallvorrichtung mit
einer Datenbank (6), die Vorlagediagnosebildinformation basierend auf verschiedenen Blickwinkeln eines lebenden Organs einer untersuchten Person speichert, wobei die Diagnosebildinformation durch ein vorbestimmtes Verfahren als Vorlage erhalten wurde,
einer Vorlagenkonvertierungseinheit (4), um durch das vorbestimmte Verfahren Tomographiebildinformation des lebenden Organs als Vorlage zu erhalten, die durch Echosignale aufgebaut wurde, die durch eine mit der untersuchten Person in Kontakt gebrachte Ultraschallsonde (1) gesammelt wurden, so dass die Vorlagetomographiebildinformation mit der in der Datenbank (6) gespeicherten Vorlagediagnosebildinformation in Beziehung gesetzt werden kann,
einer Abgleicheinheit (5) zum Abgleichen der durch die Vorlagenkonvertierungseinheit (4) als Vorlage erhaltenen Tomographiebildinformation mit der in der Datenbank (6) gespeicherten Vorlagediagnosebildinformation, und
einer Anzeigevorrichtung (9) zum Anzeigen eines Abgleichergebnisses der Abgleicheinheit (5) in Echtzeit zusammen mit Tomogrammen des lebenden Organs, die durch Echosignale aufgebaut wurden, die durch die mit der untersuchten Person in Kontakt gebrachte Ultraschallsonde (1) gesammelt wurden,
**dadurch gekennzeichnet, dass**
in dem vorbestimmten Verfahren ein Signalwertvektor $(A; A_p)$ der Diagnose- oder Tomographiebildinformation in einen Eigenraum oder einen Subraum projiziert wird, um einen Vorlagevektor $(A^*, A^*_{new}; A^*_p, A^*_{new})$ zu erhalten, und die Abgleicheinheit (5) dazu ausgelegt ist, das Abgleichergebnis des Blickwinkels mit dem Vorlagevektor $(A^*, A^*_p)$ in der gespeicherten Vorlagediagnosebildinformation zu bestimmen, die den kleinsten Unterschied zu dem von der Tomographiebildinformation erhaltenen Vorlagevektor $(A^*_{new}; A^*_{pnew})$ aufweist.

2. Vorrichtung nach Anspruch 1, wobei das lebende Organ der untersuchten Person ein Herz ist und die Tomographiebildinformation davon in Synchronisation mit einer vorbestimmten Zeitphase auf einem elektrokardiografischen Komplex der untersuchten Person erhalten wird.

3. Vorrichtung nach Anspruch 2, wobei die Abgleicheinheit (5) dazu ausgelegt ist, die einer vorbestimmten Zeitphase entsprechende Vorlagediagnosebildinformation aus der Datenbank (6) auszulesen.

4. Vorrichtung nach Anspruch 3, wobei die in der Datenbank (6) gespeicherte Vorlagediagnosebildinformation nur die Vorlagediagnosebildinformation ist, die der vorbestimmten Zeitphase entspricht.

5. Vorrichtung nach Anspruch 2, ferner mit einer Identifikationsverarbeitungs-Beurteilungseinheit zum Beurteilen, ob eine Identifikationsverarbeitung eines vorherigen Tomogramms beendet ist oder nicht.

6. Vorrichtung nach Anspruch 1, wobei ein Teil der in der Vorlagenkonvertierungseinheit (4) als Vorlage erhaltenen Tomographiebildinformation in einer derartigen Form erstellt wird, dass er in der Datenbank (6) registriert werden kann.

7. Vorrichtung nach Anspruch 6, wobei ein in die Vorlagenkonvertierungseinheit (4) eingegebenes Signal ein Signalwert von jedem Punkt ist, der längs einer Richtung eines Ultraschallstrahls von der Ultraschallsonde (1) auf einem Tomogramm abgetastet wird.

8. Vorrichtung nach Anspruch 6, wobei ein in die Vorlagenkonvertierungseinheit (4) eingegebenes Signal ein Signalwert von jedem Punkt ist, der in einer vertikalen oder einer lateralen Richtung auf einer Tomogrammebene (DL) abgetastet wird.

9. Vorrichtung nach Anspruch 6, wobei das Tomographiebild des Herzens in Synchronisation mit der Zeitphase der Bewegungsgeschwindigkeit des Herzens erhalten wird, die mit einer vorbestimmten Zeitphase auf einem elektrokardiographischen Komplex der untersuchten Person in Zusammenhang steht.

10. Vorrichtung nach Anspruch 1, wobei wenn der Abgleich zwischen der in der Abgleicheinheit (5) als Vorlage erhaltenen Tomographiebildinformation und der in der Datenbank (6) gespeicherten Vorlagediagnosebildinformation in der Abgleicheinheit (5) Übereinstimmung ergibt, eine Symbolmarkierung (12) oder ein Buchstabe (13), der die Art der Tomographiebildinformation darstellt, als ein Abgleichergebnis auf der Anzeigevorrichtung (9) angezeigt wird.

11. Vorrichtung nach Anspruch 1, wobei
als Vorlage erhaltene Diagnosebildinformation, die eine Verletzungsstelle enthält, in der Datenbank (6) zusammen mit Standardvorlagediagnosebildinformation einer gesunden Person gespeichert wird, und
wenn der Abgleich zwischen der Vorlagetomographieinformation und der in der Datenbank (6) gespeicherten Vorlagediagnosebildinformation, die die Verletzungsstelle enthält, in der Abgleicheinheit (5) Übereinstimmung ergibt, ein Krankheitsname der Tomographieinformation als ein Abgleichergebnis auf der Anzeigevorrichtung (9) angezeigt wird.

12. Vorrichtung nach Anspruch 1, wobei die in der Datenbank (6) gespeicherte als Vorlage erhaltene Diagnosebildinformation mit Zusatzinformation versehen wird, die Alter, Geschlecht und Krankheitsname der untersuchten Person enthält, sowie einen Ursprungsmesseinstellwert und einen vergangenen Messeinstellwert, die der Vorlagediagnosebildinformation zugeordnet sind.

**Revendications**

1.  Dispositif échographique comprenant :

    une base de données (6) stockant des informations d'images de diagnostic calibrées basées sur divers points de vue d'un organe vivant d'un patient, lesdites informations d'images de diagnostic ayant été calibrées par un procédé prédéterminé ;
    une unité de conversion de calibre (4) pour calibrer, au moyen dudit procédé prédéterminé, des informations d'images tomographiques de l'organe vivant construites par des signaux d'écho collectés par une sonde à ultrasons (1) mise en contact avec le patient, de sorte que les informations d'images tomographiques calibrées peuvent être corrélées avec les informations d'images de diagnostic calibrées stockées dans la base de données (6) ;
    une unité d'appariement (5) pour apparier les informations d'images tomographiques calibrées par l'unité de conversion de calibre (4) avec les informations d'images de diagnostic calibrées stockées dans la base de données (6) ; et
    un dispositif d'affichage (9) pour afficher un résultat d'appariement de l'unité d'appariement (5) en temps réel en même temps que des tomographies de l'organe vivant construites par les signaux d'écho collectés par la sonde à ultrasons (1) mise en contact avec le patient,
    **caractérisé en ce que** :

    ledit procédé prédéterminé comprend la projection d'un vecteur de valeur de signal $(A ; A_p)$ des informations d'images de diagnostic ou tomographiques dans un espace propre ou un sous-espace pour obtenir un vecteur calibre $(A^*, A^*_{new} ; A^*_p, A^*_{pnew})$, et
    ladite unité d'appariement (5) est adaptée pour déterminer comme résultat de l'appariement le point de vue avec le vecteur calibre $(A^* ; A^*_p)$ dans les informations d'images de diagnostic calibrées stockées qui a la plus petite différence par rapport au vecteur calibre $(A^*_{new} ; A^*_{pnew})$ obtenu à partir des informations d'images tomographiques.

2.  Dispositif selon la revendication 1, dans lequel l'organe vivant du patient est un coeur, et les informations d'images tomographiques de ce dernier sont obtenues en synchronisation avec une phase temporelle prédéterminée d'un complexe électrocardiographique du patient.

3.  Dispositif selon la revendication 2, dans lequel l'unité d'appariement (5) est adaptée pour extraire de la base de données (6) les informations d'images de diagnostic calibrées correspondant à une phase temporelle prédéterminée.

4.  Dispositif selon la revendication 3, dans lequel les informations d'images de diagnostic calibrées stockées dans la base de données (6) ne sont que les informations d'images de diagnostic calibrées correspondant à la phase temporelle prédéterminée.

5.  Dispositif selon la revendication 2, comprenant en outre une unité de détermination de traitement d'identification pour déterminer si un traitement d'identification d'une précédente tomographie est fini ou non.

6.  Dispositif selon la revendication 1, dans lequel une partie des informations d'images tomographiques calibrées dans l'unité de conversion de calibre (4) est mise sous une forme qui permet leur inscription dans la base de données (6).

7.  Dispositif selon la revendication 6, dans lequel un signal fourni à l'unité de conversion de calibre (4) est une valeur de signal depuis chaque point qui est balayé le long d'une direction d'un faisceau d'ultrasons émis par la sonde à ultrasons (1) sur une tomographie.

8.  Dispositif selon la revendication 6, dans lequel un signal fourni à l'unité de conversion de calibre (4) est une valeur de signal depuis chaque point qui est balayé dans une direction verticale ou latérale sur un plan tomographique (DL).

9.  Dispositif selon la revendication 6, dans lequel l'image tomographique du coeur est obtenue en synchronisation avec la phase temporelle de la vitesse de déplacement du coeur qui est associée à une phase temporelle prédéterminée sur un complexe électrocardiographique du patient.

10. Dispositif selon la revendication 1, dans lequel, quand l'appariement entre les informations d'images tomographiques calibrées dans l'unité d'appariement (5) et les informations d'images de diagnostic calibrées stockées dans la base de données (6) est satisfait dans l'unité d'appariement (5), une marque graphique (12) ou un caractère (13) représentant le type des informations d'images tomographiques est affiché(e) en tant que résultat de l'appariement sur le dispositif d'affichage (9).

**11.** Dispositif selon la revendication 1, dans lequel :

des informations d'images de diagnostic calibrées contenant un site de lésion sont stockées dans la base de données (6) en plus d'informations d'images de diagnostic calibrées standard d'une personne en bonne santé, et quand l'appariement entre les informations tomographiques calibrées et les informations d'images de diagnostic calibrées contenant le site de lésion stockées dans la base de données (6) est satisfait dans l'unité d'appariement (5), un nom de maladie des informations tomographiques est affiché en tant que résultat d'appariement sur le dispositif d'affichage (9).

**12.** Dispositif selon la revendication 1, dans lequel les informations d'images de diagnostic calibrées stockées dans la base de données (6) sont complétées avec des informations annexées contenant l'âge, le sexe et le nom de la maladie du patient, une valeur de consigne initiale de mesure et une valeur de consigne de mesure passée qui sont associées aux informations d'images de diagnostic calibrées.

# FIG.1

PROBE — 1

SIGNAL VALUE STORAGE UNIT — 2

SIGNAL VALUE EXTRACTING UNIT — 3

INPUT DEVICE — 7

TEMPLATE CONVERSION UNIT — 4

TOMOGRAM DATA APPENDING MEANS — 8

DISPLAY DEVICE — 9

MATCHING UNIT — 5

DATABASE — 6

MEASURING UNIT — 11

MEASUREMENT SETTING UNIT — 10

VIEW A    VIEW B    ...

EP 1 949 857 B1

# FIG.2

S1 — DETECT R-WAVE

S2 — PREVIOUS TOMOGRAM IDENTIFICATION PROCESSING FINISHED ? — **no**

**yes**

S3 — OBTAIN SIGNAL

S4 — CONVERT SIGNAL TO TEMPLATE

DATABASE — CORRELATE WITH DATABASE — S5

S6 — TOMOGRAM IDENTIFIED ? — **no**

**yes**

S7 — DISPLAY IDENTIFIC-ATION RESULT

S8 — MEASUREMENT SET ? — **no**

**yes**

S9 — START MEASUREMENT

S10 — DISPLAY MEAS-UREMENT RESULT

# FIG.3

EP 1 949 857 B1

FIG.4

DL

EP 1 949 857 B1

FIG.5

DL

FIG.6

DL

FIG.7

9A

DL

CARDIAC APEX
TWO-CHAMBER

12

13

# FIG.8

EP 1 949 857 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002140689 A